Europäisches Patentamt

European Patent Office

Office européen des brevets

(11) Publication number: **0 097 973**
**B1**

## EUROPEAN PATENT SPECIFICATION

(45) Date of publication of patent specification: **12.07.89**

(21) Application number: **83106388.8**

(22) Date of filing: **30.06.83**

(51) Int. Cl.⁴: **C 12 P 19/24, C 12 R 1/645**

(54) Process for preparing fructose.

(30) Priority: **30.06.82 US 393849**
**30.06.82 US 393590**
**30.06.82 US 393589**

(43) Date of publication of application:
**11.01.84 Bulletin 84/02**

(45) Publication of the grant of the patent:
**12.07.89 Bulletin 89/28**

(84) Designated Contracting States:
**AT BE CH DE FR GB IT LI LU NL SE**

(56) References cited:
**FR-A-2 215 467**

**AGRICULTURAL AND BIOLOGICAL
CHEMISTRY, vol. 42, no. 5, 1978, pages 971-
980. Y. YAMASAKI et al.: "Purification and
properties of alpha-glucosidase and
glucoamylase from Lentinus edodes (Berk.)
Sing"
CHEMICAL ABSTRACTS, vol. 91, no. 17, 22nd
October 1979, page 495, no.138821d,
Columbus, Ohio, USA. M. EZMAT EL-ZALAKI et
al.: "Edible mushrooms as producers of
amylases" & FOOD CHEM. 1979, 4(3), 203-211**

(73) Proprietor: **NABISCO BRANDS, Inc.
Nabisco Brands Plaza
Parsippany New Jersey 07054 (US)**

(72) Inventor: **Horwath, Robert O.
223 Bayberry Lane
Westport Connecticut (US)**
Inventor: **Irbe, Robert M.
41 Kingswood-Valley View Road
Norwalk Connecticut (US)**

(74) Representative: **Brauns, Hans-Adolf, Dr. rer. nat.
et al
Hoffmann, Eitle & Partner, Patentanwälte
Arabellastrasse 4
D-8000 Munich 81 (DE)**

Courier Press, Leamington Spa, England.

## Description

This invention relates to enzymatic processes for liquefying starch and converting the liquefied starch to glucose (dextrose) which can then be converted to fructose (levulose).

Most food grade glucose is provided as an enzymatic hydrolysate of corn starch, i.e., the corn syrup of commerce. Glucose is generally rated at being 60 to 80% as sweet as sucrose and therefore sells at a correspondingly lower price. It has long been known to isomerize glucose to fructose which is even sweeter than sucrose by employing an enzyme having glucose isomerase activity, preferably one which has been immobilized upon an inert support such as diethylaminoethyl-cellulose, porous glass or chitin. The isomerization of glucose provides an equilibrium mixture typically containing 42—55% fructose and is referred to as high fructose corn syrup (HFCS).

It is known that alpha-amylase, glucoamylase and glucose isomerase can be isolated from a substantial number of bacteria including species of *Streptomyces, Bacillus, Acetobacter, Norcardia, Lactobacillus, Ampullariella*, and various other genera of bacteria, and the enzymes have been employed in the commercial production of fructose from starch by known enzymatic techniques to provide mixtures of glucose and fructose. In the commercial process most commonly in present use, corn starch is liquefied, enzymatically or chemically, and then treated with glucoamylase to produce glucose which is thereafter isomerized using glucose isomerase to mixtures containing both fructose and glucose. Higher concentrations of fructose are particularly desirable and may be obtained by the use of more active enzymes and/or the use of high isomerization temperatures.

Detailed descriptions of the enzymatic conversion of glucose to fructose employing glucose isomerase can be found in Hamilton, et al. "Glucose Isomerase: a Case Study of Enzyme-Catalyzed Process Technology", *Immobilized Enzymes in Food and Microbial Processes*, Olson, et al., Plenum Press, New York, (1974), pp. 94—106, 112, 115—137; Chen, et al., "Glucose Isomerase (a Review)", *Process Biochem..*, (1980), pp. 36—41; Nordahl, et al., "Fructose Manufacture from Glucose by Immobilized Glucose Isomerase", *Chem. Abstracts*, Vol. 82, (1975), Abs. No. 11036h; and Takasaki "Fructose Production by Glucose Isomerase", *Chem. Abstracts*, vol. 81, (1974), Abs. No. 7674a. In addition, there are numerous patents relating to glucose isomerization of which U.S. Patent Nos. 3,616,221, 3,623,953 (Reissue 28,885), 3,694,313, 3,708,397, 3,715,276, 3,788,945, 3,909,354, 3,960,663, and 4,308,349 are representative.

Because of the economics involved in producing glucose isomerase, it is of the utmost importance to use the isomerase under conditions whereby maximum yields of fructose are produced using minimum quantities of glucose isomerase. Moreover, the conditions for isomerization should be such that minimal quantities of objectionable by-products are produced.

In accordance with the present invention there is provided a process for preparing fructose from glucose comprising treating starch with alpha-amylase, contacting the resulting liquefied starch with glucoamylase to hydrolyze the starch to glucose, and isomerizing at least a part of the resulting glucose to fructose by contacting the glucose with glucose isomerase characterized in that the alpha-amylase, the glucoamylase and the glucose isomerase are obtained by cultivating an organism of the *Basidiomycetes* class of fungi.

It has now been surprisingly discovered that fungi of the class *Basidiomycetes* produce significant quantities of alpha-amylase, glucoamylase and glucose isomerase. In particular, species of *Flammulina, Phellinus, Irpex Micromella, Stereum, Perenniporia, Ramaricium, Sebacina, Lentinus, Coriolus* and *Panellus* accummulate not only glucose isomerase activity which is produced in the mycelia of these organisms, but also alpha-amylase and glucoamylase which are extracellular and, therefore, accummulate in the nutrient medium. A species of *Irpex* are particularly desirable for accumulating all three enzymes. One can readily separate the glucose isomerase from the other two enzymes by merely filtering the cells. The mycelia can be employed in the enzymatic reaction mixture as a source of glucose isomerase or the enzyme activity can be separated from the mycelia by known methods after harvesting the mycelia from the media in which grown. The glucose isomerase can be separated from the mycelia by the usual extraction techniques, e.g., using sonic treatment or chemical lysing or alternatively the mycelia can be used directly. Of course, to avoid needless expense, the nutrient medium containing alpha-amylase and glucoamylase activity can be used as the source of the enzymes or, if desired, the enzymes can be separated from the nutrient medium and one from the other by using known techniques, e.g., column adsorption of the nutrient medium containing enzymes followed by selective elution of each enzyme.

In addition to the aforementioned microorganisms, the present invention contemplates the use of mutants and variants thereof as well as genetically transformed microorganisms derived therefrom by introduction of the enzyme genes into other microorganisms, including mesophilic and preferably thermophilic microorganisms. In addition, the isolated genes can be mutated to improve the properties of the respective enzymes with which they are associated. For example, the glucose isomerase gene can be mutated. The mutated glucose isomerase genes selected for such use are those which provide glucose isomerase which is stable at elevated temperatures, especially above 90°C and preferably up to about 110°C. Such genes can be prepared by the usual techniques used for mutation of microorganisms such as irradiation or chemical means. Thus, isolated glucose isomerase genes which produce glucose isomerase of moderate thermal stability, upon *in vitro* mutagenesis will undergo mutation, and selection of the appropriate mutated genes is accomplished by reintroduction of the mutated gene into either the parent or other organism, preferably a thermophilic organism followed by replication of the organism and testing of the thermal stability of the resulting glucose isomerase.

In the present invention, starch is liquefied by the action of alpha-amylase, hydrolyzed by employing glucoamylase to obtain glucose, and the glucose is enzymatically isomerized by glucose isomerase to fructose. The three enzymes utilized herein are produced by the same microorganism of the class *Basidiomycetes* particularly *Irpex mollis*.

The starch starting material of this invention is obtained from cereal grains such as corn, milo, wheat, rye and the like and amylaceous roots and tubers such as potatoes, yams, carrots, cassava (manioc), and the like. In the United States, corn starch is especially preferred due to its comparatively low cost and ready availability.

Since all three enzymes may be produced by the same microorganism, fructose can be obtained from starch in a single batch reactor by the mere expediency of adding the *Basidiomycetes* mycelia to the reactor in which the alpha-amylase and glucoamylase conversion were carried out or in a separate reactor after glucoamylase conversion is completed.

Starch can be liquefied with alpha-amylase by any of the art-recognized procedures. Since the production of food grade glucose favors the use of enzymatic starch hydrolysis procedures, such procedures are preferred herein.

Liquefied starch prepared by conventional means, such as those described in U.S. Patent Nos. 3,654,081 and 3,663,369, is preferably converted to glucose by enzymatic hydrolysis using glucoamylase produced by the microorganisms of the class *Basidiomycetes*. The glucose formed is thereafter converted to fructose by glucose isomerase produced by the same organism which produced the glucoamylase, i.e., the *Basidiomycetes*.

The liquefied starch may be obtained from cereal grains such as corn, milo, wheat, rye, and the like, and amylaceous roots and tubers such as potatoes, yams, carrots, cassava (manioc), and the like. In the United States, corn starch is especially preferred due to its comparatively low cost and ready availability. Since the production of food grade glucose favors the use of enzymatic starch hydrolysis procedures, such procedures are preferred herein. The liquefied starch is hydrolyzed to glucose by glucoamylase by procedures known to those familiar with the art. For example, the hydrolysis generally occurs at a somewhat lower temperature than the liquefication of starch, e.g., within the range of 55°C to 60°C, at a pH between 4.0 and 5.0, with 0.3—1.0% of glucoamylase (based on the weight of starch) and for about 15 to 75 hours to provide a glucose-containing solution of a high level of purity, e.g., 97—98% of glucose.

The glucose which is isomerized to fructose in accordance with the present invention can be derived from any of the known sources for this sugar. For reasons of economy, the glucose will usually be derived from the hydrolysis of starch or cellulose employing acid and/or enzyme, preferably the latter, in accordance with known procedures. Glucose obtained in this way will typically contain minor quantities of polysaccharides, sugar oligomers, etc., depending upon the carbohydrate source employed and the hydrolysis method utilized.

In the United States, corn starch is especially preferred due to its comparatively low cost and ready availability. Since the production of food grade glucose favors the use of enzymatic starch hydrolysis procedures, such procedures are preferred herein. Enzyme hydrolysis methods are described in U.S. Patent Nos. 4,017,363, 3,912,590, 3,922,196, 3,922,197—201 and 4,284,722, the disclosures of which are incorporated by reference herein. Glucose can be isomerized to fructose in accordance with the present invention employing any of the known procedures, including contacting glucose solutions with whole cells, or passing the solutions through a bed containing bound, or immobilized, glucose isomerase. Materials and procedures used for the immobilization of enzymes are well known and are described in a number of publications including Wang, et al., *Fermentation & Enzyme Technology*, John Wiley & Sons, Inc., New York (1979), pp. 318—318 and Kirk-Othmer, *Encyclopedia of Chemical Technology*, 3rd Ed., John Wiley & Sons, Inc., New York, (1980) vol. 9, pp. 148—172, the disclosures of which are incorporated by reference herein.

Particularly preferred species of the aforesaid alpha-amylase, glucoamylase and glucose isomerase producing *Basidiomycetes* for use in the present invention is *Irpex mollis*, ATCC No. 20634. Cultures of strains of this preferred species of fungi have been deposited with the American Type Culture Collection where the organism was accorded the indicated accession number, i.e., ATCC number.

Particularly preferred species of the aforesaid glucoamylase and glucose isomerase producing *Basidiomycetes* for use in the present invention include:

| Fungus | ATCC Number |
|---|---|
| *Stereum striatum* | 20633 |
| *Irpex mollis* | 20634 |
| *Lentinus edodes* | 20635 |
| *Perenniporia compacta* | 20636 |
| *Ramaricium albofdanescens* | 20637 |
| *Sebacina calcea* | 20638 |
| *Coreolus versicolor* | 20639 |
| *Panellus stipticus* | 20640 |

Cultures of strains of these preferred species of fungi have been deposited with the American Type Culture Collection where these organisms were accorded the indicated accession number, i.e., ATCC numbers.

Particularly preferred species of the aforesaid glucose isomerase producing *Basidiomycetes* for use in the present invention include:

| Fungus | ATCC Number |
|---|---|
| *Stereum striatum* | 20633 |
| *Irpex mollis* | 20634 |
| *Lentinus edodes* | 20635 |
| *Perenniporia compacta* | 20636 |
| *Ramaricium albofdanescens* | 20637 |
| *Sebacina calcea* | 20638 |
| *Coreolus versicolor* | 20639 |
| *Panellus stipticus* | 20640 |
| *Mucronella aggregata* | 20641 |
| *Flammulina velutipes* | 20642 |
| *Phellinus torulosus* | 20632 |

Cultures of strains of these preferred species of fungi have been deposited with the American Type Culture Collection where these organisms were accorded the indicated accession number, i.e., ATCC number.

The determination of other alpha-amylase, glucoamylase and glucose-isomerase producing fungi of the *Basidiomycetes* class can be carried out using simple test procedures. Cultures of the test organism are incubated for 7 days at 25°C with vigorous shaking in a growth medium containing cornsteep liquor, magnesium sulfate, potassium phosphate, xylose and agar in shake flasks. The cells are then separated from the nutrient medium by known methods, e.g., filtration, and the nutrient medium is tested for alpha-amylase, glucoamylase and glucose isomerase activity by simply adding starch to one portion of the nutrient medium and testing for liquefied starch, and adding liquefied starch to another portion of the nutrient medium and testing for the presence of glucose. The filtered mycelia are then checked for the desired enzyme activity by analysis of the enzymatic reaction solutions produced with the various substrates and determination of the presence of, for example, glucose or fructose. Fructose is identified by standard determination methods such as the acid carbazol-cysteine test or xylulose determination methods using gas chromatography or high pressure liquid chromatography.

Using these test procedures, or obvious modifications thereof, various species of fungi can be tested to determine the presence of the desired enzyme activities.

The selected fungi can be grown in accordance with known methods of propagation. One such method employs xylose as the carbohydrate source as well as other ingredients usually present in such media such as cornsteep liquor, inorganic salts and the like.

After growth for a sufficient period of time, e.g., to about 120 hours, the mycelia are harvested usually by filtration followed by washing with water buffered to a pH in the range of 6 to 7. The nutrient medium is reserved for alpha-amylase and glucoamylase activity and, if desired, the enzymes may be extracted from the nutrient medium. The glucose isomerase is then extracted from the mycelia. A convenient method of extraction involves sonic treatment or cell homogenization of aqueous mycelia suspensions in the presence of glass beads with cooling. The extracts formed can be purified employing standard techniques such as column chromatography. The enzyme extract can now be used in the isomerization reaction. Alternatively, as previously mentioned, the mycelia can be used as the source of the enzyme in the isomerization mixture.

Example I
Preparation of alpha-amylase, glucoamylase and glucose isomerase:

*Irpex mollis*, ATCC. 20634, was grown in accordance with the following:

A. Culture maintenance: After incubating the cultures on malt agar slants for 7 days at 30°C, the isolates were inoculated into shaker flasks or maintained under refrigeration (about 10°C).

B. Shake flask propagation: Inoculation medium was made up as follows:

| Ingredient | % By weight |
|---|---|
| Cornsteep liquor | 2.0 (d.b.) |
| Xylose | 5.0 |
| KH$_2$PO$_4$ | 0.1 |
| MgSO$_4$ . 7H$_2$O | 0.15 |
| Agar | 0.4 |
| adjust pH to 6.5 | |

80 ml aliquots of the above medium were placed in 500 ml Erlenmeyer flasks together with 20 ml of a 25% glucose solution (sterilized) for the inoculum fermentation and the flasks were brought to 80% of their volume with water purified by reverse osmosis. Production flasks were similarly charged except no agar was added.

First stage (test tube) propagation

In a sterile hood, approximately one half of the mycelia from a slant is transferred with a metal loop to a test tube with 10 ml of the inoculation medium and about six 3 mm glass beads (sterile). The tubes are vortexed for 30—60 seconds or until the mycelia are disrupted. The tubes are then placed on a G-50 shaker at 200 rpm, 30°C, for 7 days.

Second stage (inoculum) propagation

After 7 days, 5 ml are transferred to a 500 ml Erlenmeyer shake flask, and 1 ml is transferred into brain heart infusion to check sterility. These inoculation flasks are placed on a G-50 shaker at 200 rpm, 30°C, for 7 days.

### Third stage (production) propagation

After 7 days, 5 ml are transferred from the inoculation flask to several fermentation flasks. The fermentation flasks are placed on the G-50 shaker at 200 rpm, 30°C, for 9 days.

C. Harvesting cell biomass: After the 9-day incubation period, the pH of each shake flask was measured; the cell biomass was filtered and washed twice with pH 7.0 phosphate buffer. After the second filtration, the harvested cell biomass from each culture was weighed and frozen for bioconversion.

The nutrient medium containing alpha-amylase and glucoamylase was reserved and the enzymes were used in this form for the enzymatic conversion of starch to glucose.

### Liquefaction of starch and conversion of glucose:

An aqueous slurry of corn starch containing a water-soluble calcium compound (molarity of from about 0.003 to about 0.03) was heated to gelatinize the starch. Thereafter, the mixture was cooled to about 50—55°C, filtered nutrient medium was then added and the mixture was maintained at about 50°C until all the starch was liquefied and saccharified. The resulting mixture was maintained at this state until the desired D.E. was reached.

### Isomerization of glucose to fructose:

Whole-cell bioconversion: (under sterile conditions).

Approximately 1 gram wet weight cells is placed into a 300 ml baffled flask containing 50 ml of glucose phosphate buffer (1% glucose added to the phosphate buffer w/v) and the suspension made 0.02 M in NaF. The flask is placed on the G-50 shaker at 200 rpm, and samples are taken at 6, 12, and 24 hours by aseptically transferring 2 ml from the bioconversion flask to 15 ml Corning centrifuge tubes. The samples are centrifuged for 5 min then, 1 ml is removed and passed through a Sep-Pak $C_{18}$ cartridge (Waters Associates, Milford Ma.) following which the filtrates were analyzed by high pressure liquid chromatography (HPLC).

Bioconversion by cell-free extracts: Mycelia (4 g wet weight) in phosphate buffer (pH 6.5) are blended in a Waring blender at low speed for 15 seconds. The buffered homogenate is then transferred to a 50 ml glass Duran Sample Flask containing 50 g (about 80% by volume) glass beads of a diameter of 0.45 to 0.5 mm. The chamber is then vigorously agitated with a Braun Mechanical Cell for 1 minute while cold carbon dioxide is allowed to flow past the chamber to minimize heating.

Alternatively, the low speed blended mycelia in buffer is placed in a plastic centrifuge tube in an ice bath and then sonicated with a Heat Systems Ultrasonics Cell Disrupter, Model 350, set at 50% duty cycle, output control at 6, continuous mode, in 5 cycles of 15 seconds on and 15 seconds off.

The filtered enzyme solution was used in the isomerization reaction.

The isomerization mixture containing 50 mmoles of glucose (maleate buffered to pH 6.7), $MgCl_2$ (10 mM), $Co^{+2}$ (1 mM) and enzyme solution (50 mg of protein) was incubated at 40°C for 12 to 18 hours.

Assay of the mixture, actually aliquots thereof, showed the presence of fructose in addition to glucose. The assays employed were gas chromatography and the cysteine carbazole reaction.

Example II
Preparation of glucoamylase and glucose isomerase:

*Lentinus edodes* ATCC 20635 was grown according to the following:

A. Culture maintenance: After incubating the cultures on malt agar slants for 7 days at 30°C, the isolates were inoculated into shaker flasks or maintained under refrigeration (about 10°C).

B. Shake flask propagation: Inoculation medium was made up as follows:

| Ingredient | % By weight |
| --- | --- |
| Cornsteep liquor | 2.0 (d.b.) |
| Xylose | 5.0 |
| $KH_2PO_4$ | 0.1 |
| $MgSO_4 . 7H_2O$ | 0.15 |
| Agar | 0.4 |

80 ml Aliquots of the above medium were placed in 500 ml Erlenmeyer flasks together with 20 ml of a 25% glucose solution (sterilized) for the inoculum fermentation and the flasks were brought to 80% of their volume with water purified by reverse osmosis. Production flasks were similarly charged except no agar was added.

### First stage (test tube) propagation

In a sterile hood, approximately one half of the mycelia from a slant is transferred with a metal loop to a test tube with 10 ml of the inoculation medium and about six 3 mm glass beads (sterile). The tubes are vortexed for 30—60 seconds or until the mycelia are dispersed. The tubes are then placed on a G-50 shaker at 200 rpm, 30°C, for 7 days.

### Second stage (inoculum) propagation

After 7 days, 5 ml are transferred to a 500 ml Erlenmeyer shake flask, and 1 ml is transferred into brain heart infusion to check sterility. These inoculation flasks are placed on a G-50 shaker at 200 rpm, 30°C, for 7 days.

### Third stage (production) propagation

After 7 days, 5 ml are transferred from the inoculation flask to several fermentation flasks. The fermentation flasks are placed on the G-50 shaker at 200 rpm, 30°C, for 9 days.

C. Harvesting cell biomass: After the 9-day incubation period, the pH of each shake flask was measured; the cell biomass was filtered and washed twice with pH 7.0 phosphate buffer. After the second filtration, the harvested cell biomass from each culture was weighed and frozen for bioconversion.

The nutrient medium containing the glucoamylase was dialyzed against maleate buffer 50 mm, pH 6.7, plus CaCl$_2$ 10 mm, for 15 hours at 40°C with 3 changes.

Hydrolysis of liquefied starch to glucose:

Liquefied starch, prepared in accordance with U.S. Patent No. 4,017,363 was hydrolyzed to glucose by employing the glucoamylase obtained above at about 60°C at pH 4.5.

Isomerization of glucose to fructose:

Whole-cell bioconversion: (under sterile conditions).

Approximately 1 gram wet weight cells is placed into a 300 ml baffled flask containing 50 ml of glucose phosphate buffer (1% glucose added to the phosphate buffer w/v) and the suspension made 0.02 in NaF. The flask is placed on the G-50 shaker at 200 rpm, and samples are taken at 6, 12 and 24 hours by aseptically transferring 2 ml from the bioconversion flask to 15 ml Corning centrifuge tubes. The samples are centrifuged for 5 min the 1 ml is removed and passed through a Sep-Pak C$_{18}$ cartridge (Waters Associates, Milford Ma.) following which the filtrates were analyzed by high pressure liquid chromatography (HPLC).

Bioconversion by cell-free extracts: Mycelia (4 g wet weight) in phosphate buffer (pH 6.5) are blended in a Waring blender at low speed for 15 seconds. The buffered homogenate is then transferred to a 50 ml glass Duran Sample Flask containing 50 g (about 80% by volume) glass beads of a diameter of 0.45 to 0.5 mm. The chamber is then vigorously agitated with a Braun Mechanical Cell for 1 minute while cold carbon dioxide is allowed to flow past the chamber to minimize heating.

Alternatively, the low speed blended mycelia in buffer is placed in a plastic centrifuge tube in an ice bath and then sonicated with a Heat Systems Ultrasonics Cell Disrupter, Model 350, set at 50% duty cycle, output control at 6, continuous mode, in 5 cycles of 15 seconds on and 15 seconds off.

The extracts were filtered free of the mycelia. The isomerization mixture containing 10% of glucose (maleate buffered to pH 6.7), MgCl$_2$ (10 mM), Co$^{+2}$ (1 mM) and enzyme solution (50 mg of protein) obtained above was incubated at 60°C for 3 hours.

The reaction mixture was then heated in a boiling water bath and the precipitated protein removed by centrifugation.

Assay of the mixture, actually aliquots thereof, showed the presence of fructose in addition to glucose. The assays employed were gas chromatography and the cysteine carbazole reaction.

Example III
Preparation of glucose isomerase:

*Mucronella aggregata* ATCC 20641 was grown in accordance with the following procedure:

A. Culture maintenance: After incubating the cultures on malt agar slants for 7 days at 30°C, the isolates were inoculated into shaker flasks or maintained under refrigeration (about 10°C).

B. Shake flask propagation: Inoculation medium was made up as follows:

| Ingredient | % By weight |
|---|---|
| Cornsteep liquor | 2.0 (d.b.) |
| Xylose | 5.0 |
| KH$_2$PO$_4$ | 0.1 |
| MgSO$_4$ . 7H$_2$O | 0.15 |
| Agar | 0.4 |
| adjust pH to 6.5 | |

80 ml Aliquots of the above medium were placed in 500 ml Erlenmeyer flasks together with 20 ml of a 25% glucose solution (sterilized) for the inoculum fermentation. Production flasks were similarly charged except no agar was added.

First stage (test tube) propagation

In a sterile hood, approximately one half of the mycelia from a slant is transferred with a metal loop to a test tube with 10 ml of the inoculation medium and about six 3 mm glass beads (sterile). The tubes are vortexed for 30—60 seconds or until the mycelia are dispersed. The tubes are then placed on a G-50 shaker at 200 rpm, 30°C, for 7 days.

Second stage (inoculum) propagation

After 7 days, 5 ml are transferred to a 500 ml Erlenmeyer shake flask, and 1 ml is transferred into brain heart infusion to check sterility. These inoculation flasks are placed on a G-50 shaker at 200 rpm, 30°C, for 7 days.

Third stage (production) propagation

After 7 days, 5 ml are transferred from the inoculation flask to several fermentation flasks. The fermentation flasks are placed on the G-50 shaker at 200 rpm, 30°C, for 9 days.

C. Harvesting cell biomass: After the 9-day incubation period, the pH of each shake flask was measured; the cell biomass was filtered and washed twice with pH 7.0 phosphate buffer. After the second filtration, the harvested cell biomass from each culture was weighed and frozen for bioconversion experiments.

Cell-free extract preparation:

Mycelia (4 g wet weight) in phosphate buffer (pH 6.5) are blended in a Waring blender at low speed for 15 seconds. The buffered homogenate is then transferred to a 50 ml glass Duran Sample Flask containing 50 g (about 80% by volume) glass beads of a diameter of 0.45 to 0.5 mm. The chamber is then vigorously agitated with a Braun Mechanical Cell for 1 minute while cold carbon dioxide is allowed to flow past the chamber to minimize heating.

Alternatively, the low speed blended mycelia in buffer is placed in a plastic centrifuge tube in an ice bath and then sonicated with a Heat Systems Ultrasonics Cell Disrupter, Model 350, set at 50% duty cycle, output control at 6, continuous mode, in 5 cycles of 15 seconds on and 15 seconds off.

Isomerization of glucose to fructose:

The isomerization mixture containing 10% by weight glucose (maleate-buffered to pH 6.7), $MgCl_2$ (10 mM), $Co^{+2}$ (1 mM) and enzyme solution (50 mg of protein) was incubated at 60°C for 3 hours.

Assay of the mixture, actually aliquots thereof, showed the presence of fructose in addition to glucose. The assays employed were gas chromatography and the cysteine carbazole method (N. E. Lloyd, *Cereal Chem.*, 49, #5, pp. 544—553, 1972).

## Claims

1. The process for preparing fructose from glucose comprising treating starch with alpha-amylase, contacting the resulting liquefied starch with glucoamylase to hydrolyze the starch to glucose, and isomerizing at least a part of the resulting glucose to fructose by contacting the glucose with glucose isomerase characterized in that the alpha-amylase, the glucoamylase and the glucose isomerase are obtained by cultivating an organism of the *Basidiomycetes* class of fungi.

2. The process of Claim 1 wherein the glucoamylase and alpha-amylase activities are present in the nutrient medium employed for the growth of the organism.

3. The process of Calim 1 wherein the glucose isomerase activity is present in the mycelia of the organism.

4. The process of Claim 1 wherein the organism is a species of *Irpex*.

5. The process of Claim 4 wherein the organism is *Irpex mollis*.

6. The process for preparing fructose comprising contacting liquefied starch with glucoamylase to hydrolyze the starch to glucose and contacting the glucose so produced with glucose isomerase to isomerize at least a part of the glucose to fructose characterized in that the glucoamylase and glucose isomerase are obtained by cultivating an organism of the *Basidiomycetes* class of fungi.

7. The process of Claim 6 wherein the organism is a species of the genera *Stereum, Irpex, Lentinus, Perenniporia, Ramaricium, Sebacina, Coriolus* and *Pannellus*.

8. The process of Claim 7 wherein the organism is of the group *Stereum striatum, Irpex mollis, Lentinus edodes, Perenniporia compacta, Ramaricium albofdanescens, Sebacina calcea, Coriolus versicolor*, and *Pannellus stipticus*.

9. The process for preparing fructose comprising contacting an aqueous solution of glucose with glucose isomerase characterized in that the glucouse isomerase is obtained by cultivating an organism of the *Basidiomycetes* class of fungi to convert at least a portion of the glucose to fructose.

10. The process of Claim 9 wherein the organism is a species of the genera *Stereum, Irpex, Lentinus, Perenniporia, Ramaricium, Sebacina, Cariolus, Panellus, Mucronella, Flammulina*, and *Phellinus*.

11. The process of Claim 10 wherein the species is of the group *striatum, Irpex mollis, Lentinus edodes, Perenniporia compacta, Ramaricium albofdanescens, Sebacina calcea, Coriolus versicolor, Panellus stipticus, Mucronella aggregata, Flammulina velutipes*, and *Phellinus torulosus*.

## Patentansprüche

1. Verfahren zur Herstellung von Fructose aus Glucose, bei dem man Stärke mit alpha-Amylase behandelt, die entstandene, verflüssigte Stärke mit Glucoamylase in Berührung bringt und die Stärke zu Glukose hydrolysiert und wengistens einen Teil der erhaltenen Glucose zu Fructose isomerisiert, indem man die Glucose mit Glucoseisomerase in Berührung bringt, dadurch gekennzeichnet, dass die alpha-Amylase, die Glucoamylase und die Glucoseisomerase erhalten wurden, indem man einen Organismus der Pilzklasse Basidiomycetes kultiviert.

2. Verfahren gemäss Anspruch 1, bei dem die Glucoamylase- und alpha-Amylaseaktivitäten in dem verwendeten Nährmittel für das Wachstum des Organismus vorhanden sind.

3. Verfahren gemäss Anspruch 1, bei dem die Glucoisomeraseaktivität in den Mycelia des Organismus vorhanden ist.

4. Verfahren gemäss Anspruch 1, bei dem der Organismus eine Spezies von Irpex ist.

5. Verfahren gemäss Anspruch 4, bei dem der Organismus Irpex mollis ist.

6. Verfahren zur Herstellung von Fructose, bei dem man verflüssigte Stärke mit Glucoamylase kontaktiert und die Stärke zu Glucose hydrolysiert und die so hergestellte Glucose mit Glucoseisomerase in Berührung bringt und dadurch wenigstens einen Teil der Glucose zu Fructose isomerisiert, dadurch gekennzeichnet, dass die Glucoseamylase und Glucoseisomerase durch Kultivieren eines Organismus der Pilzklasse Basidiomycetes erhalten wurden.

7. Verfahren gemäss Anspruch 6, bei dem der Organismus eine Spezies der Genera Stereum, Irpex, Lentinus, Perenniporia, Ramaricium, Sebacina, Coriolus und Pannellus ist.

8. Verfahren gemäss Anspruch 7, bei dem der Organismus zur Gruppe Stereum striatum, Irpex mollis, Lentinus edodes, Perenniporia compacts, Ramaricium albofdanescens, Sebacina calcea, Coriolus versicolor und Pannellus stipticus gehört.

9. Verfahren zur Herstellung von Fructose, bei dem man eine wässrige Lösung von Glucose mit Glucoseisomerase in Berührung bringt, dadurch gekennzeichnet, dass die Glucoseisomerase erhalten wurde durch Kultivieren eines Organismus der Pilzklasse Basidiomycetes, und man dabei wenigstens einen Teil der Glucose in Fructose umwandelt.

10. Verfahren gemäss Anspruch 9, bei dem der Organismus eine Spezies der Genera Stereum, Irpex, Lentinus, Perenniporia, Ramaricium, Sebacina, Cariolus, Panellus, Mucronella, Flammulina und Phellinus ist.

11. Verfahren gemäss Anspruch 10, bei dem die Spezies zur Gruppe Striatum, Irpex mollis, Lentinus edodes, Perenniporia compacta, Ramaricium albofdanescens, Sebacina calcea, Coriolus versicolor, Panellus stipticus, Mucronella aggregata, Flammulina velutipes und Phellinus toruloses gehört.

**Revendications**

1. Procédé de fabrication de fructose à partir du glucose, consistant à traiter de l'amidon avec de l'alpha-amylase, à mettre en contact l'amidon liquéfié résultant avec de la glucoamylase afin d'hydrolyser l'amidon en glucose, et à isomériser au moins une partie du glucose résultant en fructose par la mise en contact du glucose avec de la glucose isomérase, caractérisé par le fait que l'alpha-amylase, la glucoamylase et la glucose isomérase sont obtenues par culture d'un organisme de la classe de champignons *Basidiomycetes*.

2. Procédé selon la revendication 1, dans lequel les activités glucoamylase et alpha-amylase sont présentes dans le milieu nutritif employé pour la croissance de l'organisme.

3. Procédé selon la revendication 1, dans lequel l'activité glucose isomérase est présente dans les mycéliums de l'organisme.

4. Procédé selon la revendication 1, dans lequel l'organisme est une espèce d'*Irpex*.

5. Procédé selon la revendication 4, dans lequel l'organisme est *Irpex mollis*.

6. Procédé de préparation de fructose consistant à mettre en contact de l'amidon liquéfié avec de la glucoamylase pour hydrolyser l'amidon en glucose et à mettre en contact le glucose ainsi obtenu avec de la glucose isomérase pour isomériser au moins une partie du glucose en fructose, caractérisé par le fait que la glucoamylase et la glucose isomérase sont obtenues par culture d'un organisme de la classe de champignons *Basidiomycetes*.

7. Procédé selon la revendication 6, dans lequelle l'organisme est une espèce des genres *Stereum, Irpex, Lentinus, Perenniporia, Ramaricium, Sebacina, Coriolus et Pannelus*.

8. Procédé selon la revendication 7, dans lequel l'organisme appartient au groupe *Stereum striatum, Irpex mollis, Lentinus edodes, Perenniporia compacts, Ramaricium albofdanescens, Sebacina calcea, Coriolus versicolor*, et *Pannelus stipticus*.

9. Procédé de préparation de fructose consistant à mettre en contact une solution aqueuse de glucose avec de la glucose isomérase, caractérisé par le fait que la glucose isomérase est obtenue par culture d'un organisme de la classe de champignons *Basidiomycetes*, pour convertir au moins une fraction du glucose en fructose.

10. Procédé selon la revendication 9, dans lequel l'organisme est une espèce des genres *Stereum, Irpex, Lentinus, Perenniporia, Ramaricium, Sebacina, Coriolus, Pannelus, Mucronella, Flammulina*, et *Phellinus*.

11. Procédé selon la revendication 10 dans lequel l'espèce appartient au groupe *Stereum striatum, Irpex mollis, Lentinus edodes, Perenniporia compacta, Ramaricium albofdanescens, Sebacina calcea, Coriolus versicolor, Panellus stipticus, Mucronella aggregata, Flammulina velutipes*, et *Phellinus torulosus*.